**Europäisches Patentamt**

**European Patent Office**

**Office européen des brevets**

(19)

(11) Veröffentlichungsnummer: **0 039 388**
**A2**

(12) # EUROPÄISCHE PATENTANMELDUNG

(21) Anmeldenummer: **81100971.1**

(22) Anmeldetag: **12.02.81**

(51) Int. Cl.³: **G 01 N 33/50, A 61 K 31/505**

(30) Priorität: **02.05.80 DE 3016818**

(43) Veröffentlichungstag der Anmeldung: **11.11.81**
**Patentblatt 81/45**

(84) Benannte Vertragsstaaten: **AT CH DE FR GB IT LI NL SE**

(71) Anmelder: **Röhm Pharma GmbH,**
**Dr.-Otto-Röhm-Strasse 2-4, D-6108 Weiterstadt 1 (DE)**

(72) Erfinder: **Mutschler, Ernst, Prof.Dr., Rechenberg 24,**
**D-6500 Mainz-Hechtsheim (DE)**
Erfinder: **Völger, Karl-Dieter, Dr., Bebelstrasse 10,**
**D-6101 Bickenbach (DE)**
Erfinder: **Rölz, Wolfgang, Dr., Carl-Schurz-Strasse 55,**
**D-6070 Langen-Neurott (DE)**

(54) **Diagnostisches Verfahren zur Bestimmung der Leberfunktion.**

(57) Die Erfindung betrifft ein diagnostisches Verfahren zur Bestimmung der Leberfunktion, bei dem man bei Patienten mit vermuteter Leberfunktionsstörung 2,4,7-Triamino-6-phenylpteridin (TA) oral verabreicht und in geeignetem zeitlichen Abstand das Verhältnis von nativem TA zu dessen Phasen-II-Metaboliten (Schwefelsäurehalbester) im Plasma und/oder im Urin bestimmt, wobei ein Verhältnis natives TA zu seinem Phasen-II-Metabolit von 1:1 bis 1:(<1) im Plasma bzw. Urin das Vorliegen einer Leberfunktionsstörung anzeigt, sowie für das diagnostische Verfahren geeignete, orale Verabreichungsformen des TA.

ACTORUM AG

Diagnostisches Verfahren zur Bestimmung
der Leberfunktion

Die Funktionsdiagnostik bestimmter Körperorgane wie des Gehirns, des Herzens, der Schilddrüse, der Lunge, der Niere und nicht zuletzt der Leber ist eine wesentliche Voraussetzung für die Beurteilung der gesundheitlichen Gesamtsituation des Menschen. Die Leber ist bekanntlich das vielseitigste Stoffwechselorgan des Körpers; fast alle im Darm anfallenden und resorbierten Verdauungsspaltprodukte sowie auch Pharmaka gelangen in die Leber, um hier verstoffwechselt und zu Ausscheidungsprodukten abgebaut zu werden.

Der Rolle der Leber im Stoffwechselgeschehen entsprechend hat sich die medizinische Forschung immer wieder um die Entwicklung möglichst einfacher und in ihrer Aussage eindeutiger Leberfunktionstests bemüht, die zudem eine möglichst geringe Belastung des Organismus darstellen sollen (vgl. Ullmanns Encyklopädie der Technischen Chemie, 4. Auflage, Band 10, S. 64-84, Verlag Chemie 1975). Sehr intensiv vorangetrieben wurde die Verwendung von Radiodiagnostika, insbesondere in der Funktionsdiagnostik. Das Prinzip besteht z.B. darin, mit einem Szintillationszähler nach intravenöser Injektion einer radioaktiven Verbindung, die vom Organ ausgeschieden wird, nicht nur die Zunahme der Radioaktivität, sondern auch deren Abnahme zu messen. Die gebräuchlichsten Leberfunktionstests sind der Bromthaleintest sowie der Galaktose-Eliminationstest. Beide Testverfahren sind mit Risiken bzw. Belastungen des Patienten verbunden, so daß diese nicht mehr oder nur selten angewandt werden. Es bestand daher die Notwendigkeit nach weiteren einfacheren diagnostischen Verfahren zu suchen, die den Funktionszustand der Leber bestimmen, z.B. im Zustand der Leberzirrhose.

Es wurde gefunden, daß sich Einschränkungen der Leberfunktion mit sehr geringem Aufwand und mit großer diagnostischer und analytischer Sicherheit nachweisen lassen, wenn man 2,4,7-Triamino-6-phenylpteridin (Triamteren = TA) dem Patienten oral verabreicht und in geeignetem zeitlichen Abstand das Verhältnis TA zu OH-TA-Ester im Plasma und/oder im Urin des Patienten bestimmt. Bei normaler Leberfunktion beträgt das Verhältnis TA zu OH-TA-Ester 1:3 bis 1:10. Je nach Ein- schränkung der Leberfunktion beträgt das Verhältnis TA zu OH-TA-Ester 1:1 bis 1: ( < 1). Man findet demnach je nach Einschränkung der Leberfunktion mehr natives TA als OH-TA-Ester, was als eine direkte Auswirkung der gestörten Funktion der Leber zu metabolischer Umwandlung von körperfremden Stoffen im Organismus und damit als Indikator für die Leberfunktion überhaupt gewertet werden kann. Außerdem wurde festgestellt, daß bei Leberzirrhotikern in der Regel die Plasma-Halbwertszeiten (zur Bestimmung der Plasmahalbwertszeit siehe Seite 5) von TA und OH-TA-Ester deutlich erhöht, beispielsweise verdoppelt sind.

Triamteren ist ein seit langem bekanntes Antikaliuretikum, dessen Angriffspunkt vorrangig an der Niere liegt, jedoch sind neuerdings auch kardiale Wirkungen beobachtet worden. Triamteren wird vorteilhaft in Kombination mit anderen Diuretika, insbesondere Saluretika, zur Vermeidung von Kaliumverlusten therapeutisch eingesetzt.

Die Verabreichung des Triamteren zur erfindungsgemäßen Bestimmung der Leberfunktionseinschränkung geschieht oral. Bei der Herstellung der Verabreichungsformen (Kapseln, Tabletten etc.) und der Verabreichung selbst, kann von den Erfahrungen mit Triamteren als Therapeutikum ausgegangen werden. Die zur Durchführung des erfindungsgemäßen diagnostischen Verfahrens benötigten Mengen an Triamteren können innerhalb gewisser Grenzen schwanken, beispielsweise zwischen 50 und 300 mg, vorzugsweise bis 100 mg. Zur Einhaltung standardisierter Bedingungen kann es zweckmäßig sein, gleichbleibende Quantitäten an Triamteren zu verab-

- ·4 -

reichen. Dabei kann vorteilhaft von bereits auf dem Markt befindlichen Handelsformen (z. B. Kapseln mit 50 mg Triamteren Jatropur$^R$) Gebrauch gemacht werden.

Vorteilhafterweise bedient man sich bei der Durchführung des Tests spezifischer, zur oralen Verabreichungsformen geeigneten, Zubereitungen, die zur raschen Freisetzung und biologischen Verfügbarmachung des Triamterens führen.
Eine besonders geeignete Ausführungsform besteht in einer Tablettenformulierung gemäß Beispiel.

Mit dieser Tablettenformulierung wird eine stets gleichbleibende in vivo-Freisetzung gewährleistet, was wesentlich zur Funktionstüchtigkeit des Tests beiträgt.

Die Belastung des Organismus der Patienten ist dabei gering. Im Falle des Vorliegens einer Leberzirrhose mit Ascites weist Triamteren überdies einen in der Therapie dieser Erkrankung mit gutem Erfolg angewandten therapeutischen Effekt auf.

Als Kontrolle der diagnostischen Sicherheit der erfindungsgemäßen Methode wurden bei Vergleichsversuchen die folgenden Parameter der Leberfunktion herangezogen:

Das Rotochem-Profil, das die Bestimmung der Glutamatoxalacetat-transaminase (GOT), die Glutamat-pyruvat-

- ·5 -

transaminase (GPT), die Gamma-glutamyl-transpeptidase (GGT), die alkalischen Phosphatasen (AP) und das Gesamt-Bilirubin einschließt, weiter die Serum-elektrophorese, der Normotest, der ein Maß für die Gerinnung, insbesondere für den Prothrombin-Gehalt darstellt und die Galaktose-Eliminations-Kapazität. Zusätzlich wurde von jeder Versuchsperson das Blutbild untersucht.

Bei dem Test wurde darauf geachtet, daß die Versuchspersonen der gleichen Altersgruppe und dem gleichen Geschlecht angehörten und eine annähernd gleiche Nierenfunktion vorlag. Während der Versuchsperiode waren sie stationär aufgenommen und unterlagen denselben Ernährungsbedingungen. Außer den unumgänglich notwendigen, wurden alle anderen Medikamente, insbesondere Diuretika vor Versuchsbeginn abgesetzt.

Bei diesen Untersuchungen wurde festgestellt, daß die Konzentration des OH-TA-Esters bei lebergesunden Versuchspersonen in Plasma um das vier- bis zwölffache höher liegt als die der nativen Substanz Triamteren (TA). Die Plasmahalbwertszeiten wurden nach der Formel $t_{50\%} = \dfrac{\ln 2}{k\,2}$ berechnet, die Flächen unter den Kurven konnten mit Hilfe der Trapezregel errechnet werden.

Die Durchführung des erfindungsgemäßen diagnostischen Verfahrens kann ebenfalls mit dem Urin der Patienten erfolgen. Während einer Versuchsdauer von 5 Tagen konnte TA und der OH-TA-Ester nachgewiesen werden.

## Ergebnisse

Das Verhältnis des OH-TA-Esters zu TA lag bei Lebergesunden zwischen (4-10):1, bei Leberzirrhotikern zwischen (1-3,2):1 (bei einem Zirrhotiker kehrte sich sogar das Verhältnis um).

Die Plasmahalbwertszeiten für TA und OH-TA-Ester, die bei Lebergesunden bei etwa 5,5 Stunden liegen, betragen bei Leberzirrhotikern durchschnittlich 11,2 Stunden. Die Plasmahalbwertszeiten sind daher auf das Doppelte verlängert.

Die Ausscheidung von TA und OH-TA-Ester im Harn ist bei Lebergesunden nach 72 Stunden beendet, bei Leberzirrhotikern ist sie dagegen nach 5 Tagen immer noch nicht völlig abgeschlossen.

Die Pteridinaabkömmlinge TA und OH-TA-Ester zeichnen sich durch eine leichte analytische Erfassbarkeit aus. Sie beruht darauf, daß beide bei UV-Bestrahlung eine hellblaue Fluoreszenz zeigen, die noch in sehr geringen Konzentrationen wahrnehmbar ist.

Die analytische Bestimmung kann nach B. Grebian et al. Arzneim.-Forsch./Drug Res. 26, 2125-2127 (1976) erfolgen. Die Plasmaproben werden mit dem doppelten Volumen Methanol verdünnt, das ausfallende Protein ausgefällt. Vom Überstand wird ein definierter

- 7 -

Anteil auf Kieselgel-Platten (z. B. Kieselgel-60-Fertigplatten "Merck") ohne Fluoreszenzindikator aufgetragen. Anschließend wird mit dem Fließmittel Essigsäureethylester/Methanol/Ammoniak 25 %ig (60 + 30 + 10) bei Kammersättigung chromatographiert. Danach werden die Platten getrocknet und die Substanzen fluoreszenzspektroskopisch gemessen.

Analog kann auch die Konzentration von TA und OH-TA-Ester im Urin bestimmt werden.

Die erfindungsgemäß besonders bevorzugten Zubereitungen in Tablettenform sind dadurch charakterisiert, daß der Wirkstoff Triamteren zunächst in ein per se leichtlösliches Teilgranulat überführt und dieses Teilgranulat in einem weiteren Schritt zusammen mit zerfallsbeschleunigenden Hilfsstoffen zu schnellzerfallenden Tabletten verpresst wird.

Als Hilfsstoffe für das leichtlösliche Teilgranulat kommt z. B. in Frage Milchzucker, Cellulose, einschlägig verwendete Stärketypen, kolloidale Kieselsäure sowie leichtlösliche Bindemittel, z. B. Polyvinylpyrrolidon.

- ·8 -

Als zerfallsbeschleunigenden Hilfsstoff für das Teilgranulat haben sich Alkali- und Erdalkalicarbonate bewährt; genannt sei beispielsweise Calciumcarbonat.

Die Herstellung des Teilgranuläts kann im ersten Schritt durch trockenes Mischen der vorgenannten Bestandteile und anschließendes Überfeuchten und Rücktrocknen durch Zugabe eines der obengenannten Hilfsstoffe vorgenommen werden.

Trocknung des auf diese Weise hergestellten Materials und nachfolgende teilchengrößenkontrollierte Zerkleinerung ergibt ein Granulat mit den oben beschriebenen Eigenschaften.

Zur Herstellung der erfindungsgemäßen Tabletten wird eine Pulvermischung hergestellt, die anschließend verpresst wird.

Die Pulvermischung kann z. B. Füllstoffe der auch für das Teilgranulat anwendbaren Art enthalten. Zusätzlich enthält sie zerfallsfördernde Agentien, wie Salze der Carboxymethylcellulose, Polyvinylpyrrolidon u. ä.

Als Tablettierhilfsstoffe können z. B. Magnesiumstearat und/oder Talkum eingesetzt werden.

Die Verpressung der Tablette kann z. B. auf Excenter- oder Rundläuferpressen bei in weiten Bereichen variierbaren Pressdrucken vorgenommen
werden. Dadurch wird die erwünschte, kurze Zerfallszeit von etwa 1 min nicht beeinträchtigt.

Zur Erläuterung der Herstellung der erfindungsgemäßen, schnellzerfallenden Tabletten soll das
folgende Beispiel dienen:

1a) Herstellung des Triamteren-Teilgranulats für
einen 50 kg- Ansatz:

|  | kg |
|---|---|
| Calciumcarbonat | 3,450 |
| Triamteren | 23,150 |
| Aerosil® | 0,220 |
| Maisstärke | 7,900 |
| Lactose K | 7,000 |
| Milchzuckergranulat | 7,580 |
| Kollidon 25 | 0,700 |
| Isopropanol | 9,500 |
| Wasser gereinigt | 3,000 |

Kollidon 25, Isopropanol, Wasser gereinigt: Granulierflüssigkeit

- 10 -

b) Herstellung der Tablettenmasse für einen
100 kg-Ansatz:

In einem Taumelmischer werden das Teilgranulat gemäß 1a) mit folgenden Hilfsstoffen vermischt:

|                        | kg         |
|------------------------|------------|
| Calcium-CMC            | 2,000      |
| Triamteren-Granulat    | 36,000     |
| Aerosil $^R$           | 0,340      |
| Maisstärke             | 0,900      |
| Magnesiumstearat       | 0,500      |
| Talkum                 | 5,600      |
| Milchzuckergranulat EE | 54,560     |
|                        | 100,000 kg |

c) Verpressung der Tablettenmasse gemäß 1b) zu
leichtzerfallenden Tabletten.

Auf einer Rundläuferpresse werden bei einem
Pressdruck von 1,0 bis 2,0 ton/cm$^2$ Tabletten
vom Durchmesser 10 mg, Gewicht 300 mg und
einer Härte von 4 - 6 Kp Schleuniger-Härte
hergestellt.

Diagnostisches Verfahren zur Bestimmung
der Leberfunktion

Patentansprüche

1. Diagnostisches Verfahren zur Bestimmung
   der Leberfunktion bei Probanden,

   dadurch gekennzeichnet,

   daß man bei Patienten mit vermuteter Leberfunktionseinschränkung 2,4,7-Triamino-6-
   phenyl-pteridin (Triamteren = TA) oral verabreicht und in geeignetem zeitlichen Abstand
   das Verhältnis von nativem Triamteren zu seinem
   Phase-II-Metaboliten, dem Schwefelsäurehalbester des 2,4,7-Triamino-6-(p-hydroxyphenyl)-
   pteridin (OH-TA-Ester) im Plasma und/oder im
   Urin des Patienten bestimmt, wobei ein Verhältnis natives Triamteren (TA) zu seinem
   Phase-II-Metaboliten (OH-TA-Ester) von 1:1
   bis 1: (< 1) im Plasma bzw. Urin das Vorliegen
   einer Leberfunktionseinschränkung anzeigt,
   während ein Verhältnis TA zu OH-TA-Ester von
   1:3 bis 1:10 im Plasma bzw. Urin ein Indiz
   für eine normale Leberfunktion darstellt.

2. Diagnostisches Verfahren gemäß Anspruch 1, dadurch gekennzeichnet, daß man 50 - 300 mg, vorzugsweise 100 mg TA, oral verabreicht.

3. Orale Verabreichungsformen für 2,4,7-Triamino-6-phenyl-pteridin, die zur raschen Freisetzung des Wirkstoffs führen als diagnostisches Mittel zur Bestimmung der Leberfunktion gemäß Anspruch 1 und 2.

4. Orale Verabreichungsform gemäß Anspruch 3 in Tablettenform, dadurch gekennzeichnet, daß man das 2,4,7-Triamino-6-phenylpteridin zunächst in ein per se leichtlösliches Teilgranulat überführt und dieses Teilgranulat in einem weiteren Schritt zusammen mit zerfallbeschleunigenden Hilfsstoffen zu schnellzerfallenden Tabletten verpreßt.

5. Orale Verabreichungsform gemäß Anspruch 4, dadurch gekennzeichnet, daß das Teilgranulat infolge eines Gehalts an carbonhaltigen Hilfsstoffen nach Art einer Brausetablette zerfällt.